# EUROPEAN PATENT SPECIFICATION

(11) **EP 1 718 249 B1**
(45) Date of publication and mention of the grant of the patent: **27.04.2011**
(21) Application number: 04782223.4
(22) Date of filing: 25.08.2004
(51) Int. Cl.: A61F 2/24, A61M 25/10

(54) **DEVICES FOR ENDOVASCULAR MITRAL VALVE CORRECTION FROM THE LEFT CORONARY SINUS**
VORRICHTUNGEN FÜR DIE ENDOVASKULÄRE MITRALKLAPPENKORREKTUR VOM LINKEN KORONARSINUS
DISPOSITIFS DE CORRECTION ENDOVASCULAIRE DE LA VALVULE MITRALE A PARTIR DU SINUS CORONAIRE GAUCHE

(30) Priority: 25.02.2004 US 787574
(43) Date of publication of application: 08.11.2006
(73) Proprietor: Edwards Lifesciences Corporation, Irvine, CA 92614 (US)
(72) Inventor: ZARBATANY, David, Laguna Niguel, CA 92677 (US); SCHRECK, Stefan, Vista, CA 92083 (US)
(74) Representative: Parry, Christopher Stephen
(86) International application number: PCT/US2004/027692
(87) International publication number: WO 2005/082288

(56) References cited:
- WO-A-02/078576

## Description

### FIELD

This invention relates to an apparatus for heart valve repair an d, more particularly, to endovascular apparatus for improving mitral valve function using devices inserted into the left coronary sinus.

### BACKGROUND

Mitral valve repair is the procedure of choice to correct mitral regurgitation of all etiologies. With the use of current surgical techniques, between 70% and 95% of regurgitant mitral valves can be repaired. The advantages of mitral valve repair over mitral valve replacement are well documented. These include better preservation of cardiac function and reduced risk of anticoagulant-related hemorrhage, thromboembolism and endocarditis.

In current practice, mitral valve surgery requires an extremely invasive approach that includes a chest wall incision, cardiopulmonary bypass, cardiac an d pulmonary arrest, and an incision on the heart itself to gain access to the mitral valve. Such a procedure is associated with high morbidity and mortality. Due to the risks associated with this procedure, many of the sickest patients are denied the pote ntial benefits of surgical correction of mitral regurgitation. In addition, patients with moderate, symptomatic mitral regurgitation are denied early intervention and undergo surgical correction only after the development of cardiac dysfunction.

Mi tral regurgitation, or leakage from the outflow to the inflow side of the valve, is a common occurrence in patients with heart failure and a source of morbidity and mortality in these patients. Mitral regurgitation in patients with heart failure is caused by changes in the geometric configurations of the left ventricle, papillary muscles and mitral annulus. These geometric alterations result in mitral leaflet tethering and incomplete coaptation at systole. In this situation, mitral regurgitation is correcte d by plicating the mitral valve annulus, either by (i) sutures alone or by (ii) sutures in combination with a support ring, so as to reduce the circumference of the distended annulus and restore the original geometry of the mitral valve annulus.

More particularly, current surgical practice for mitral valve repair generally requires that the posterior mitral valve annulus be reduced in radius by surgically opening the left atrium and then fixing sutures, or sutures in combination with a support ring, to the internal surface of the annulus; this structure is used to pull the annulus back into a smaller radius, thereby reducing mitral regurgitation by improving leaflet coaptation.

This method of mitral valve repair, generally termed "annuloplast y", effectively reduces mitral regurgitation in heart failure patients. This, in turn, reduces symptoms of heart failure, improves quality of life and increases longevity. Unfortunately, however, the invasive nature of mitral valve surgery and the attendant risks render most heart failure patients poor surgical candidates. Thus, a less invasive means to increase leaflet coaptation and thereby reduce mitral regurgitation in heart failure patients would make this therapy available to a much greater percentage of patients.

Several recent developments in minimally invasive techniques for repairing the mitral valve without surgery have been introduced by several different companies. Mitralife of Santa Rosa, CA proposes various systems for remodeling the mitral annulus utilizing elongated structures that are percutaneously introduced into the left coronary sinus and re -shape the mitral annulus therefrom. The left coronary sinus is that blood vessel commencing at the coronary ostia in the left atrium and passing through the atrioventricular groove in close proximity to the posterior, lateral and medial aspects of the mitral annulus. Because of its position adjacent the mitral annulus, the coronary sinus provides an ideal conduit for positioning an endovascular prosthesis to act on the mitral annulus and therefore re -shape it. Mitralife discloses in PCT publication WO 02/060352 and related applications a number of elongated devices that either cinch or otherwise reduce the size of the mitral annulus from the coronary sinus. Because of the complex pathway to and through the coronary sinus, the elongated devices are designed to have a first configuration for delivery and may assume a second configuration within the coronary sinus to cause reshaping of the mitral annulus. For example, an elongated tube having a natural tendency to bend is straightened with a guidewire, passed into the coronary sinus, and then the guidewire is removed to permit the tube to bend in a desired manner. Alternatively, a shape memory material may be utilized. Viacor, Inc. of Wilmington MA presents similar systems in PCT publication WO 02/078576 (closest prior art), and related applications. The devices shown in the Viacor publications are primarily designed to straighten the natural curvature of at least a portion of the coronary sinus in the vicinity of the posterior leaflet of the mitral valve so that the posterior annulus displaces in an anterior direction. In one embodiment, Viacor proposes utilizing a balloon to provide the straightening force within the coronary sinus.

Despite recent attempts at minimally invasive repair of the mitral annulus using devices residing in the left coronary sinus, there is a need for such endovascular correction devices that are less traumatic to the sinus and also more reliable over the long-term.
Further, there is a need for better control over the shape in which the mitral annulus is deformed by such endovascular correction devices.

### SUMMARY

The present invention provides an improved catheter-based device for reshaping a mitral valve annulus.

Accordingly, the invention provides a system for endovascular mitral valve correction, comprising: a catheter having a balloon assembly; characterised by the balloon assembly including a first balloon having a length and a second balloon also having a length, the catheter having separate lumens connected to inflate the first and second balloons, the balloon assembly including the first and second balloons having a total length and opposed ends, wherein at least a first opposed end of the balloon assembly is flexible so as to permit bending relative to an adjacent portion of the balloon assembly. The catheter is inserted into the vasculature of the systems of the patient and advanced such that the balloon assembly is located within the left coronary sinus. Subsequently, the first and second balloons are inflated.

In one embodiment, the second balloon is arranged concentrically around the first balloon and has a length that is greater than the first balloon, the second balloon being mounted such that opposed ends thereof extend beyond opposed ends of the first balloon. In another alternative embodiment, the first and second balloons are arranged in series along the balloon assembly and are connected to each other by a portion of the catheter that permits relative bending therebetween.

In yet another embodiment of the invention, a system for endovascular mitral valve correction includes a catheter having a balloon thereon having a length, the balloon having a balloon wall including a mid -section contiguous with opposed end sections, the mid -section being formed differently than at least a first end section so as to be more rigid than the first end section when the balloon is inflated.

In another embodiment of the invention, a system for endovascular mitral valve correction includes a catheter having a balloon thereon, the balloon adapted to be positioned within a coronary sinus and adapted to remodel a mitral valve annulus adjacent to the coronary sinus, the balloon containing a hardenable material to maintain the balloon in an inflated conditions in the coronary sinus.

In all of the embodiments mentioned herein the catheter may include at least one inflation lumen and one-way valve into an interior of the balloon or balloons. In addition, a mechanism may be provided for decoupling the balloon catheter from the balloon assembly in order to maintain the balloon assembly within the coronary sinus on a relatively long -term basis.

### BRIEF DESCRIPTION OF THE DRAWINGS

Figure 1 is a schematic plan view from above of the mitral valve, left coronary sinus, and adjacent aortic valve;

Figures 2a and 2b are plan views of alternative mitral valve reshaping balloons of the present invention in their inflated configurations;

Figures 3a -3c illustrate a portion of a distal end of a catheter of the present invention having a reshaping balloon thereon and a one-way fill valve;

Figure 4 shows the reshaping balloon of Figure 2a deployed in the coronary sinus;

Figure 5 shows the reshaping balloon of Figure 2b deployed in the coronary sinus;

Figure 6a is a schematic view of a mitral valve reshaping balloon assembly having first and second concentric balloons mounted on a catheter;

Figure 6b is a schematic view of the balloon assembly of Figure 6a illustr ating the flexibility of opposed ends thereof;

Figures 7a and 7b are schematic views of, respectively, a prior art linear mitral annulus reshaper and the balloon assembly of Figure 6a deployed in the coronary sinus;

Figure 8a is a schematic view of a mitral valve reshaping balloon assembly having first, second, and third balloons mounted in series on a catheter;

Figure 8b is a schematic view of the balloon assembly of Figure 8a illustrating the flexibility of opposed ends thereof;

Figure 9a is a schematic sectional view of the distal end of a mitral valve reshaping balloon catheter of the present invention illustrating a step of using an inflation catheter to inflate the balloon through a one-way valve; and

Figure 9b is a schematic sectional view of the balloon catheter of Figure 9a after removal of the inflation catheter from within the balloon assembly, thus permitting de -coupling of the catheter from the reshaping balloon.

### DESCRIPTION OF THE PREFERRED EMBODIMENTS

The present application describes a number of improvements over catheter-based devices of the prior art for reshaping a mitral valve annulus by inserting the devices into the left coronary sinus. These devices are first inserted into the vascular system of the patient through a number of well -known techniques, including percutaneously via the jugular vein, and advanced through vasculature such that a distal end thereof is within the left coronary sinus. The term "distal end" to describe placement of the various annulus reshapers on the catheter is used for convenience, and a portion of the catheter or other elongated device used to position the reshaper may extend beyond the reshaper to the actual distal end of the delivery device. Therefore, "distal end" in this sense means distal along the catheter with respect to a proximal end which is outside the body. At the same time, the term catheter is representative of any elongated tube or implement that can be used to deliver the reshaping tool through the vas culature to the coronary sinus.

A number of embodiments described herein include inflation balloons for reshaping the mitral annulus. The balloons may be filled with saline or other biocompatible fluid. One aspect of the present invention is to fill the reshaping balloons with a fluid that can subsequently harden into a solid or semi -solid. For example, the fluid may cure or cross -link over time or upon exposure to heat or other stimulus. A polymeric composition such as a silicone resin, e.g., an RTV, or a 2-part epoxy resin, is suitable in this regard. Alternatively, the fluid may be of a type that can be cross-linked so as to convert from a liquid form to a solid form upon the addition of a catalyst. For example, a first fluid, such as an acrylic type resin, or a silicone resin, may be used to inflate the balloon and then a second cross -linking fluid may be added to harden the first fluid. Another potential technique included in the present invention is to harden a fluid, such as an ultraviolet light (UV) curable polymer using energy such as light delivered through a probe having a light delivery tip thereon. The probe would be advanced into proximity with the inflated balloon, or inserted to be within the interior of the balloon. These structures and techniques for converting a liquid into a solid or semi-solid will not be described in further detail herein, though it will be understood that the spectrum of such structures or techniques are covered herein.

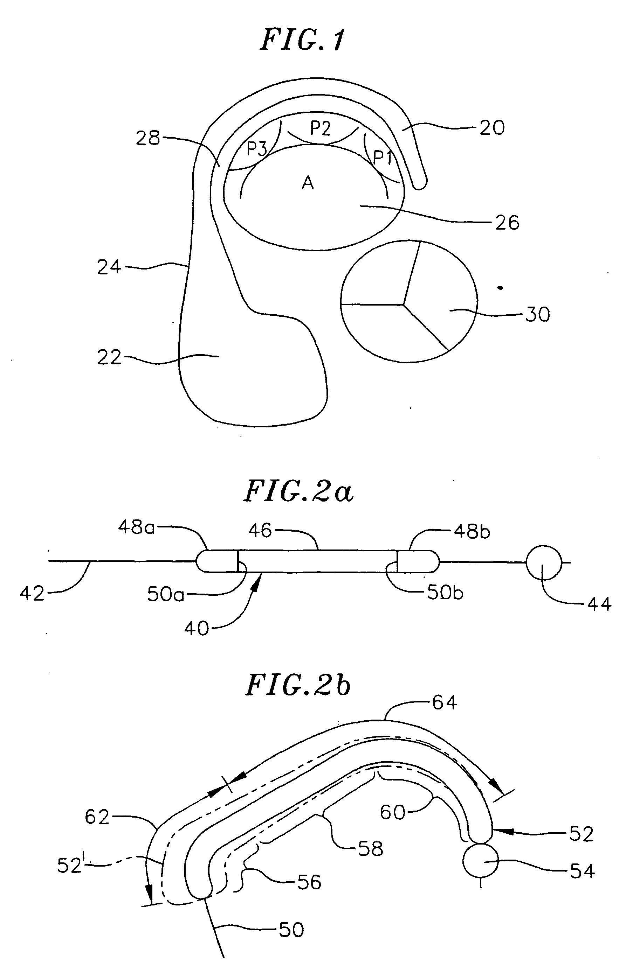

With reference to Figure 1, the left coronary sinus 20 extends from the right atrium 22 and coronary ostia 24 and wraps around the mitral valve 26. The mitral annulus 28 is that portion of tissue surrounding the mitral valve orifice to which the several leaflets attach. The mitral valve 26 is described as having two leaflets -- an anterior leaflet A and a posterior leaflet made up of three scallops P1, P2, P3. The general position of the adjacent aortic valve 30 is shown for orientation.

The problem of mitral regurgitation of ten results from the posterior aspect of the mitral annulus 28 dilating so as to displace one or more of the posterior leaflet scallops P1, P2, or P3 away from the anterior leaflet A. To reduce or eliminate mitral regurgitation, therefore, it is desirable to move the posterior aspect of the mitral annulus 28 in an anterior direction. For instance, in the specific case of ischemic mitral regurgitation, the posterior section of the mitral valve dilates asymmetrically, and predominantly in the region of the P3 scallop. Consequently, it is desirable to move the area of the mitral annulus 28 adjacent the P3 scallop more toward the center of the mitral valve 26 while leaving the remaining section of the mitral annulus unaltered. The catheter-based devices of the present invention can be inserted within the coronary sinus 20 to the proper location so as to perform the desired, reshaping procedure on the mitral annulus 28.

Figure 2a illustrates a first embodiment of a catheter-based mitral annulus -reshaping device of the present invention that includes a balloon 40 mounted on or near the distal end of a catheter 42.
A second balloon 44 for anchoring the reshaping balloon 40 within the coronary sinus 20 is provided, typically distally on the catheter with respect to the reshaping balloon, as shown. The reshaping balloon 40 comprises a mid -section 46 contiguous with a pair of opposed end sections 48a, 48b. Continuous in this sense means that the balloon 40 has a single -layered balloon wall along its entire length such that the mid -section 46 and end sections 48a, 48b define a continuous tube connected at joint lines 50a, 50b. The mid -section 46 has a different construction than the end sections 48a, 48b so as to be more rigid when the balloon 40 is inflated. For example, the mid -section 46 may have a greater wall thickness than the end sections 48a, 48b, or may be impregnated with a tubular matrix of fabric or the like. Alternatively, the mid -section 46 may be a different material tha n the end sections 48a, 48b, the two materials being joined at the lines 50a, 50b such as with co-extrusion, adhesives, or ultrasonic welds. A combination of polymers such as nylon, PEBAX, PET, polyethylene (different durometers) can also be used, such as by laminating or co -extruding the multiple materials. When the balloon 40 is inflated, the mid - section 46 provides a relatively rigid, linear reshaping portion while the end sections 48a, 48b are capable of flexing or bending to conform to the curvilinear coronary sinus 20 and reduce damage thereto.

Figure 2b illustrates a second embodiment of a mitral annulus -reshaping device having a catheter 50 on which a hook -shaped or otherwise curvilinear balloon 52 is mounted. The balloon 52 is delivered in a deflated state and assumes the illustrated shape upon inflation. Again, a second, typically smaller anchoring balloon 54 may be utilized in conjunction with the reshaping balloon 52. In the specific embodiment illustrated, the reshaping balloon 52 inc ludes a curvilinear proximal end section 56, a generally linear mid-section 58, and a curvilinear distal end section 60. The end sections 56, 60 may have different shapes, for example, the proximal end section 56 is shorter than the distal end section 60. Likewise, the mid-section 58 may be other than linear, and there may be more than three discrete sections along the reshaping balloon 52. It will therefore be appreciated that an inflatable reshaping device can take an infinite number of forms and may be customized to correct particular pathologies within the diseased mitral valve.

Desirably, the balloon 52 when inflated is tapered as depicted in phantom at 52' in Fig. 2b such that the proximal end section 56 has a greater outer diameter (OD) tha n the distal end section 60. On average, the coronary sinus inner diameter (ID) tapers down from about 15 mm at the proximal coronary ostia to about 5 mm at the distal end. The OD of the balloon 52 should therefore match (or at least correlate with) the sinus ID at the implant location. The balloon 52 may be undersized slightly to permit blood flow there around. Therefore, for example, a balloon 52 that extends the entire length may have an OD of about 15 mm at the proximal end section 56 and an OD of about 5 mm at the distal end section 60. In other exemplary configurations, shorter balloons may taper from 6 -4 mm OD, or from 15 -10 mm OD, depending on the placement. These diameter ranges apply to all of the embodiments in the present application. In the illustrated embodiment, a proximal length 62 of the balloon 52' has a relatively steep taper while a distal length 64 has a more gradual taper. This configuration is believed to most accurately mimic the average coronary sinus shape.

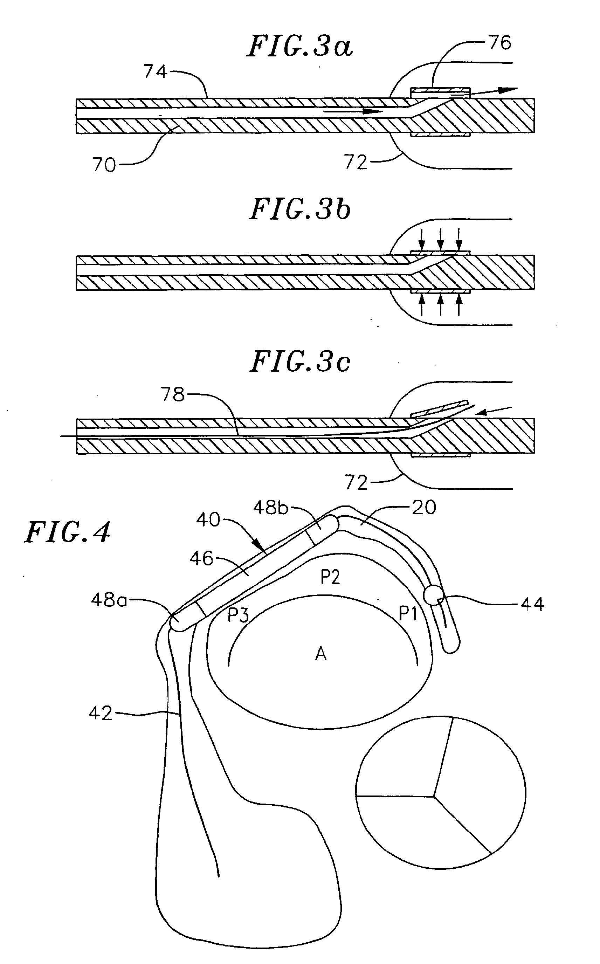

Figures 3a -3c illustrate a particular configuration during several steps of inflation of any of the balloons of the present invention. A balloon catheter 70 carries the reshaping balloon 72 on its distal end. An inflation lumen 74 opens through a one - way valve 76 into the interior of the balloon 72. The one-way valve 76 in the illustrated embodiment comprises an elastic sleeve that is easily displaced radially outward upon fluid pressure within the lumen 74, as shown in Figure 3a. The balloon 72 may be made from high-pressure resistant polymer, such as polyethylene terephthalate (PET), which is capable of the withstanding pressures of up to 400 psi. When inflated to such pressures, the balloon 72 becomes relatively rigid and maintains its preformed shape. Also, the high-pressure within the balloon 72 acts on the exterior surface of the one -way valve 76, as seen in Figure 3b, and prevents deflation of the balloon. In case of complications, or other need to deflate the balloon 72, a guidewire 78 or other such prob e may be passed through the lumen 74 as seen in Figure 3c so as to displace the one-way valve 76 outward or puncture the one way valve 76 and relieve pressure from within the balloon 72.

Figure 4 illustrates the placement of the mitral annulus -reshaping device of Figure 2a, wherein the reshaping balloon 40 is inflated on or near the distal end of the catheter 42 in a predetermined position within the coronary sinus 20. Specifically, the relatively rigid linear mid -section 46 of the balloon 40 is gen erally centered in the coronary sinus next to the P3 scallop of the posterior leaflet. This placement is particularly beneficial for correcting ischemic mitral regurgitation. The relatively more flexible end sections 48a, 48b reduce potential damage or other trauma to the walls of the coronary sinus 20.

The anchoring balloon 44 is shown further along the coronary sinus from the reshaping balloon 40. Because the operation is carried out while the heart is beating, the anchoring balloon 44 helps pre vent migration of the reshaping balloon 46 prior to its inflation. The diameter of the reshaping balloon 40 is such that blood is permitted to flow around it after inflation. Alternatively, longitudinal channels or grooves can be provided in the balloon 40 to permit greater blood flow. For example, one or more spiral grooves or channels may be formed in the exterior of the balloon 40. In a further alternative, the balloon 40 may completely occlude the coronary sinus 20 such that blood no longer flows th erethrough. Some studies indicate that collateral perfusion of the heart in the absence of flow through the coronary sinus 20 is sufficient.

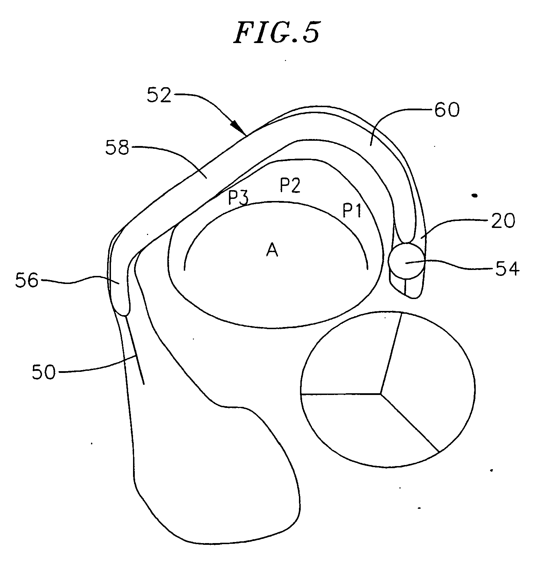

Figure 5 illustrates the placement of the curvilinear mitral annulus -reshaping device of Figure 2b, wherein the reshaping balloon 52 is inflated on or near the distal end of the catheter 50 within the coronary sinus 20. Again, the relatively linear mid - section 58 is positioned adjacent the P3 scallop of the posterior leaflet to correct for ischemic mitral regurgitation. The curvilinear end sections 56, 60 are shown conforming to the curvature of the coronary sinus 20, which helps reduce trauma thereto. Also, the anchoring balloon 54 is seen inflated just distal to the reshaping balloon 52. As before, the anc horing balloon 54 is typically deployed prior to inflation of the reshaping balloon 52.

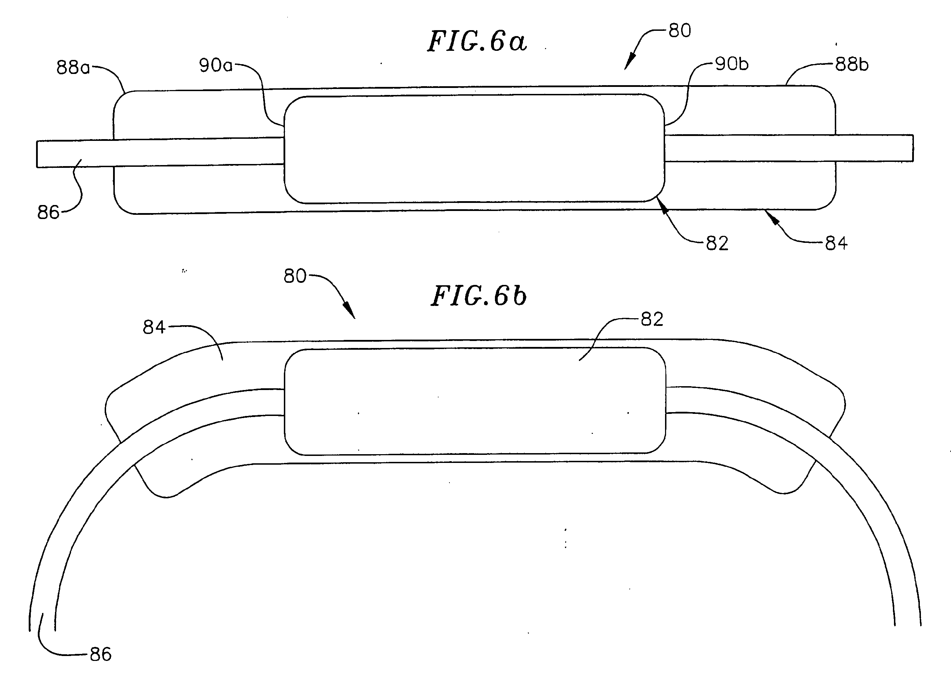

Figures 6a and 6b schematically illustrate a still further catheter -based reshaping device of the present invention. A balloon assembly 80 comprising a first balloon 82 and a second balloon 84 mounts at or near the distal end of a balloon catheter 86. Although not shown, the balloon catheter 86 includes one or more lumens for jointly or independently inflating the balloons 82, 84. The second balloon 84 is arran ged concentrically around the first balloon 82 and has a length that is greater than the first balloon. In the illustrated embodiment, the second balloon 84 is mounted such that opposed ends 88a, 88b thereof extend beyond opposed ends 90a, 90b of the firs t balloon 82. Consequently, the opposed ends 88a, 88b of the second balloon 84 define the opposed ends of the balloon assembly. The length of the balloon assembly 80 is desirably at least about one -third the length of the coronary sinus, or at least the length between the commissures of the adjacent mitral valve, transferred to the coronary sinus. In an exemplary embodiment, the length of first balloon 82 may be between about 50 -100 mm, and the second balloon 84 may be sized longer such that the opposed ends 88a, 88b overhang the first balloon by about 10 mm. Another way to look at the length ranges is that the opposed ends 88a, 88b each has a length that is between about 8 -17% of the total length of the balloon assembly 80 (both ends 88a, 88b and a first balloon 82 having a length of 100 mm, versus one end 88a and a first balloon 82 having a length of 50 mm). These length ranges apply to all of the embodiments in the present application.

Because of different materials or construction, or because the second balloon 84 is inflated to a lesser pressure than the first balloon 82, the opposed ends of the balloon assembly 80 are relatively more flexible than a mid -section thereof. The inner or first balloon 82 may be inflated to between about 3-20 atmospheres, while the second balloon 84 is inflated to between about 1 -6 atmospheres. More specifically, the mid-section of the balloon assembly 80 comprises that portion coincident with the first, inner balloon 82, and the opposed ends of the balloon assemb ly coincide with the opposed ends 88a, 88b of the second balloon 84. Figure 6b illustrates the balloon assembly 80 after inflation and after deployment within the coronary sinus (not shown), or when subjected to bending stresses. The opposed ends of the balloon assembly 80 are permitted to flex to help prevent undue trauma to the inner walls of the coronary sinus. Prior to implant, the balloon assembly 80 is desirably deflated and flattened by pulling a vacuum on both balloons 82, 84 and then wrapped and heat set within a tube to create a low delivery profile.

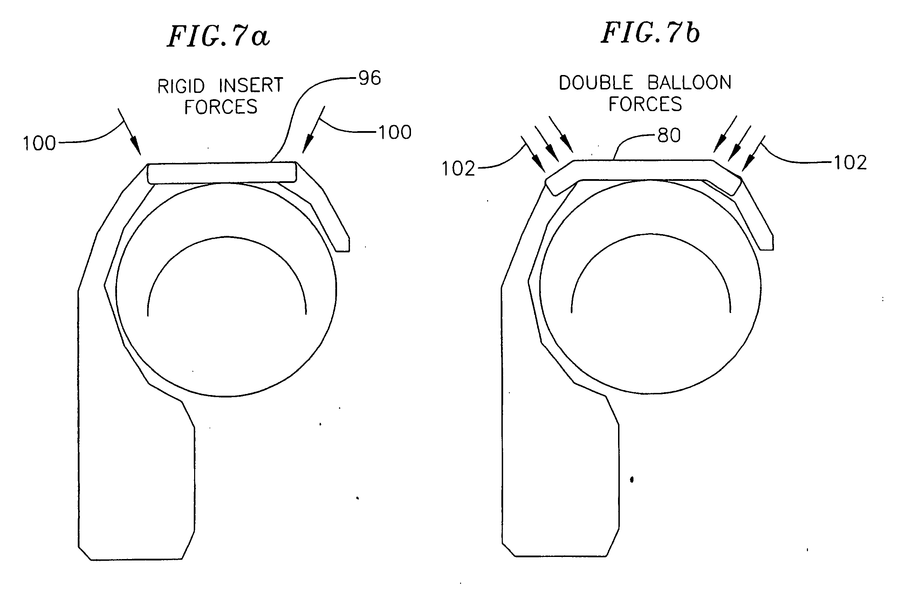

Figures 7a and 7b contrast the deployment of a linear, rigid insert 96 (Figure 7a) in the coronary sinus and a mitral valve reshaping device, such as balloon assembly 80 of Figure 6a (Figure 7b). Figure 7a illustrates the reaction forces 100 applied by the outer curve of the coronary sinus on the ends of the rigid insert 96. This concentration of reaction forces 100 tends to create points of abrasion or trauma within the coronary sinus. On the other hand, Figure 7b illustrates reaction forces 102 that are more widely distributed along the opposed ends of the balloon assembly 80. Further, the opposed ends of the balloon assembly 80 are relatively flexible and curve to conform to coronary sin us, therefore avoiding altering the adjacent mitral annulus.

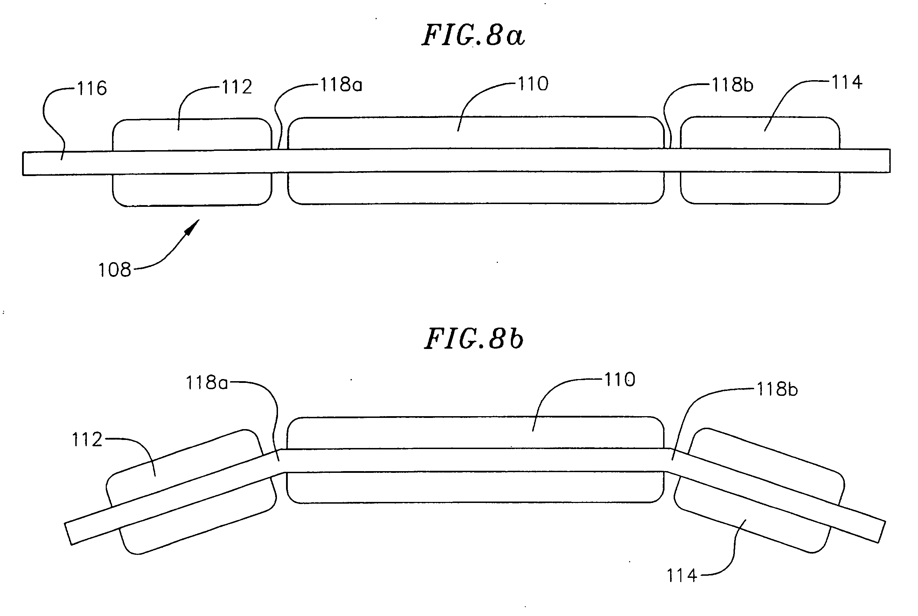

Figures 8a and 8b illustrate a still further embodiment of a mitral valve reshaping device of the present invention. In particular, a balloon assembly 108 comprises a first balloon 110, a second balloon 112, and a third balloon 114 mounted in series along a balloon catheter 116. The first balloon 110 is located in between the second and third balloons 112, 114.
Small gaps remain between the balloons 110, 112, 114 so that short portions 1 18a, 118b of the catheter 116 permit bending of the balloons relative to each other, as seen in Figure 8b. In this manner, the opposed ends of the balloon assembly 108 are rendered more flexible than a mid -section thereof, in the same manner as the concentric balloon assembly 80 of Figures 6a and 6b. The deployment of the balloon assembly 108 in the coronary sinus therefore results in less trauma to the surrounding tissue.

Preferably, the central, first balloon 110 is substantially linear and has a length greater than either of the second or third balloons 112, 114. Also, the balloon assembly 108 may be provided with only one end balloon 112 or 114, depending on the need. As mentioned above, the balloon catheter 116 may be provided with a single inflation lumen for simultaneously inflating the three balloons 110, 112, 114, or separate inflation lumens may be utilized. If separate lumens are utilized, one or more of the series of balloons, typically the central balloon 110, may be inflated to a greater pressure than the others to provide a more rigid reshaping force at that location.

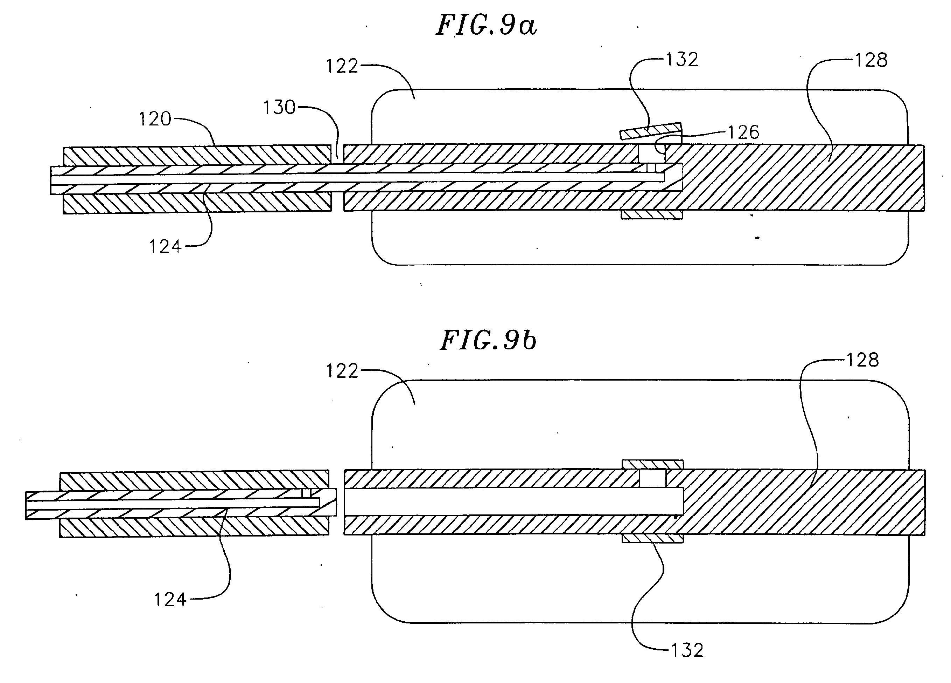

Figures 9a and 9b illustrate structure and use of an exemplary mechanism for decoupling a balloon catheter 120 from a mitral valve reshaping balloon assembly 1 22. The various devices of the present invention are intended to be implanted within the coronary sinus on a relatively long -term basis. Therefore, the delivery catheter must be removed and a mechanism for decoupling the two provided.

In the illus trated embodiment, an inflation catheter 124 extends through a lumen of the balloon catheter 120 and terminates adjacent an inflation port 126 formed in a distal extension 128 of the balloon catheter. The distal extension 128 is coextensive with, but deco upled from, the main length of the balloon catheter 120, as seen at gap 130. A one -way valve 132, such as the elastic sleeve described above, cooperates with inflation port 126 to permit inflation of the balloon assembly 122 and prohibit deflation thereof . The distal end of the inflation catheter 124 fits closely within the lumen of the extension 128, or is otherwise temporarily secured thereto during delivery and deployment of the balloon assembly 122. After inflation of the balloon assembly 122, the in flation catheter 124 is removed from within the distal extension 128, such as by proximally withdrawing the inflation catheter while holding the balloon catheter 120 stationery. Once the inflation catheter 124 is completely withdrawn from the distal exten sion 128, the balloon assembly 122 with the distal extension are decoupled from the proximal portion of the balloon catheter 120. of course, other arrangements for performing the decoupling function are contemplated within the scope of present invention.

While the foregoing describes the preferred embodiments of the invention, various alternatives, modifications, and equivalents may be used. Moreover, it will be obvious that certain other modifications may be practiced within the scope of the appended claims.

## Claims

1. A system for endovascular mitral valve correction, comprising:
a catheter (86) having a balloon assembly (80); **characterised by** the balloon assembly including a first balloon (82) having a length and a second balloon (84) also having a length, the catheter having separate lumens connected to inflate the first and second balloons, the balloon assembly including the first and second balloons having a total length and opposed ends (88a, 88b), wherein at least a first opposed end of the balloon assembly is flexible so as to permit bending relative to an adjacent portion of the balloon assembly.

2. The system of Claim 1, wherein the second balloon (84) is arranged concentrically around the first balloon (82) and has a length that is greater than the first balloon, the second balloon being mounted such that opposed ends thereof (88a, 88b) extend beyond opposed ends (90a, 90b) of the first balloon and define the opposed ends of the balloon assembly.

3. The system of Claim 1, wherein the first and second balloons (110, 112) are arranged in series along the balloon assembly and are connected to each other by a portion of the catheter (118a) that permits relative bending therebetween so that the second balloon (112) defines the first opposed end of the balloon assembly that is flexible.

4. The system of Claim 1, further including a third balloon (114) arranged in series adjacent the first balloon along the balloon assembly such that the first balloon (110) is between the second and third balloons (112, 114), the third balloon being (114) connected to the first balloon (112) by a portion of the catheter (118b) that permits relative bending therebetween so as to define a second opposed end of the balloon assembly that is flexible.

5. The system of Claim 4, wherein the second and third balloon (112, 114) each has a length that is shorter than the length of the first balloon (110).

6. The system of Claim 1, wherein the first opposed end that is flexible has a length that is between about 8-17% of the total length of the balloon assembly

7. The system of Claim 1, wherein the balloon assembly is tapered when inflated such that a proximal end (56) has a greater outer diameter than a distal end (60) thereof.

## Patentansprüche

1. Ein System für die endovaskuläre Mitralklappenkorrektur, die umfasst:
einen Katheter (86) mit einer Ballonbaugruppe (80); **gekennzeichnet dadurch, dass** die Ballonbaugruppe einen ersten Ballon (82), der eine Länge aufweist, und einen zweiten Ballon (84), der auch eine Länge aufweist, umfasst, wobei der Katheter separate, zum Aufblasen des ersten und des zweiten Ballons verbundene Lumen aufweist, wobei die Ballonbaugruppe den ersten und zweiten Ballon umfasst, die eine Gesamtlänge und entgegengesetzte Enden (88a, 88b) aufweisen, wobei mindestens ein erstes entgegengesetztes Ende der Ballonbaugruppe flexibel ist, um eine Verbiegung gegenüber einem angrenzenden Teil der Ballonbaugruppe zu erlauben.

2. Das System gemäß Anspruch 1, wobei der zweite Ballon (84) konzentrisch um den ersten Ballon (82) angeordnet ist und eine Länge aufweist, die größer ist als die des ersten Ballons, wobei der zweite Ballon so angebracht ist, dass entgegengesetzte Enden desselben (88a, 88b) sich über die entgegengesetzten Enden (90a, 90b) des ersten Ballons hinaus erstrecken und die gegenüberliegenden Enden der Ballonbaugruppe bestimmen.

3. Das System gemäß Anspruch 1, wobei der erste und zweite Ballon (110, 112) hintereinander entlang der Ballonbaugruppe angeordnet sind und durch einen Teil des Katheters (118a) miteinander verbunden sind, der eine relative Verbiegung zwischen diesen erlaubt, so dass der zweite Ballon (112) das erste entgegengesetzte Ende der Ballonbaugruppe definiert, das flexibel ist.

4. Das System gemäß Anspruch 1, das weiter einen dritten Ballon (114) umfasst, der angrenzend an den ersten Ballon in einer Reihe entlang der Ballonbaugruppe angeordnet ist, so dass der erste Ballon (110) zwischen dem zweiten und dritten Ballon (112, 114) liegt, wobei der dritte Ballon (114) durch einen Teil des Katheters (118b) mit dem ersten Ballon (112) verbunden ist, der eine relative Verbiegung zwischen diesen erlaubt, so dass er ein zweites entgegengesetztes Ende der Ballonbaugruppe bestimmt, das flexibel ist.

5. Das System gemäß Anspruch 4, wobei der zweite und dritte Ballon (112, 114) jeweils eine Länge aufweisen, die kürzer ist als die Länge des ersten Ballons (110).

6. Das System gemäß Anspruch 1, wobei das erste entgegengesetzte Ende, das flexibel ist, eine Länge aufweist, die zwischen ungefähr 8-17% der Gesamtlänge der Ballonbaugruppe liegt.

7. Das System gemäß Anspruch 1, wobei die aufgeblasene Ballonbaugruppe konisch zuläuft, so dass ein proximales Ende (56) einen größeren Außendurchmesser hat als ein distales Ende (60) derselben.

## Revendications

1. Système de correction endovasculaire de la valvule mitrale comprenant :
un cathéter (86) muni d'un ensemble ballonnet (80) ; **caractérisé par le fait que** l'ensemble ballonnet comprend un premier ballonnet (82) possédant une longueur et un deuxième ballonnet (84) possédant également une longueur, le cathéter étant doté de lumières séparées connectées de manière à gonfler les premier et deuxième ballonnets, l'ensemble ballonnet comprenant les premiers et deuxième ballonnets possédant une longueur totale et des extrémités opposées (88a, 88b), dans lequel au moins une première extrémité opposée de l'ensemble ballonnet est flexible de manière à permettre une flexion relative à une partie adjacente de l'ensemble ballonnet.

2. Système de la revendication 1, dans lequel le deuxième ballonnet (84) est disposé concentriquement autour du premier ballonnet (82) et possède une longueur qui est supérieure à celle du premier ballonnet, le deuxième ballonnet étant monté de manière telle que les extrémités opposées de celui-ci (88a, 88b) s'étendent au-delà des extrémités opposées (90a, 90b) du premier ballonnet et définissent les extrémités opposées de l'ensemble ballonnet.

3. Système de la Revendication 1, dans lequel les premier et deuxième ballonnets (110, 112) sont disposés en série le long de l'ensemble ballonnet et sont connectés l'un à l'autre par une partie du cathéter (118a), laquelle permet une courbure relative entre eux de manière à ce que le deuxième ballonnet (112) définisse la première extrémité opposée de l'ensemble ballonnet qui est flexible.

4. Système de la Revendication 1, comprenant en outre un troisième ballonnet 114) disposé en série de manière adjacente au premier ballonnet le long de l'ensemble ballonnet de manière à ce que le premier ballonnet (110) se situe entre les deuxième et troisième ballonnets (112, 114), le troisième ballonnet (114) étant connecté au premier ballonnet (112) par une partie du cathéter (118b), laquelle permet une courbure relative entre eux de manière à définir une deuxième extrémité opposée de l'ensemble ballonnet qui est flexible.

5. Système de la Revendication 4, dans lequel les deuxième et troisième ballonnets (112, 114) possèdent chacun une longueur qui est plus courte que la longueur du premier ballonnet (110).

6. Système de la Revendication 1, dans lequel la première extrémité opposée qui est flexible possède une longueur d'environ 8 à 17% de la longueur totale de l'ensemble ballonnet.

7. Système de la Revendication 1, dans lequel l'ensemble ballonnet est effilé lorsqu'il est gonflé de telle sorte qu'une extrémité proximale (56) a un diamètre extérieur supérieur à une extrémité distale (60) de celui-ci.
